## Europäisches Patentamt

## European Patent Office

## Office européen des brevets

(19)

(11) Veröffentlichungsnummer: **0 087 658**
**B1**

---

(12) **EUROPÄISCHE PATENTSCHRIFT**

---

(45) Veröffentlichungstag der Patentschrift:
**29.05.85**

(21) Anmeldenummer: **83101356.0**

(22) Anmeldetag: **12.02.83**

(51) Int. Cl.⁴: **C 07 C 2/28**, C 07 C 41/06,
C 07 C 43/04, B 01 J 31/26

---

(54) Verfahren zur katalytischen Umsetzung von i-Alken unter gleichzeitiger weitgehender Entfernung von Acetylen-Verbindungen, Carbonyl-Verbindungen und gegebenenfalls Diolefinen durch Hydrierung.

---

(30) Priorität: **26.02.82 DE 3207030**

(43) Veröffentlichungstag der Anmeldung:
**07.09.83 Patentblatt 83/36**

(45) Bekanntmachung des Hinweises auf die Patenterteilung:
**29.05.85 Patentblatt 85/22**

(84) Benannte Vertragsstaaten:
**BE DE FR GB IT NL**

(56) Entgegenhaltungen:
**EP - A - 0 028 377**
**EP - A - 0 043 986**
**EP - A - 0 048 893**

(73) Patentinhaber: **EC ERDÖLCHEMIE GMBH,**
**Postfach 75 20 02, D-5000 Köln 71 (DE)**

(72) Erfinder: **Köhler, Hans-Dieter, Dr., Stürzelberger Strasse 71, D-4047 Dormagen 5 (DE)**
Erfinder: **Scheef, Hans-Volker, Goethestrasse 69, D-4047 Dormagen 1 (DE)**
Erfinder: **Schleppinghoff, Bernhard, Dr., Adolf-Kolping-Strasse 5, D-4047 Dormagen 1 (DE)**
Erfinder: **Schulwitz, Bruno, Dr., Lützlongericher Strasse 64, D-5000 Köln 60 (DE)**

(74) Vertreter: **Mann, Volker, Dr. et al, c/o Bayer Aktiengesellschaft Zentralbereich Patente Marken und Lizenzen, D-5090 Leverkusen-Bayerwerk (DE)**

---

BUNDESDRUCKEREI BERLIN

0 087 658

## Beschreibung

Die vorliegende Erfindung betrifft ein Verfahren zur katalytischen Umsetzung von i-Alkenen unter gleichzeitiger hydrierender Entfernung von stark ungesättigten Kohlenwasserstoffen durch Umsetzung von Kohlenwasserstoffströmen, die die genannten Bestandteile enthalten, mit Wasserstoff an einem metalldotierten Kationenaustauscher.

Es ist bekannt, daß Alkyl-tert.-alkylether durch Umsetzung eines primären Alkanols mit Olefinen, die an einem tertiären Kohlenstoffatom eine Doppelbindung aufweisen, hergestellt werden können. So läßt sich beispielsweise Methanol mit Isobutylen oder Isoamylen (2-Methyl-buten-(1) bzw. -(2)) zu Methyl-tert.-butylether (MTBE) bzw. Tert.-amyl-methylether (TAME) umsetzen. Die Veretherungsreaktion ist so selektiv, daß sie sich zum Abtrennen der tert.-Olefine aus Stoffströmen, die sogar Diolefine, wie beispielsweise Butadien, enthalten, eignet (DE-OS 2 521 673, US 4 161 496). Die stärker ungesättigten Kohlenwasserstoffe, wie die $C_3$- und $C_4$-Acetylene und die Carbonyl-Verbindungen werden bei der Veretherungsreaktion nicht angegriffen. Ihre Entfernung erfolgt wahlweise durch eine separate Hydrierung oder Extraktion. So enthalten die Spaltgase der Pyrolyseprozesse in der $C_4$-Fraktion bis über 50% Butadien neben acetylenischen Verbindungen, die nach der Tieftemperaturzerlegung der Gase überwiegend bei den Olefinen mit gleicher C-Zahl verbleiben.

Zur Herstellung von polymerisationsgerechtem Butadien müssen die genannten stark ungesättigten Kohlenwasserstoffe entfernt werden. Dies geschieht beispielsweise durch katalytische Hydrierung oder Extraktion der Acetylen-Verbindungen mit geeigneten Lösungsmitteln (F. Asinger: Die petrolchemische Industrie, Akademie-Verlag, Berlin 1971, Seiten 363 bis 369).

Die Entfernung kleiner Mengen von Ethylacetylen und Vinylacetylen aus der $C_4$-Fraktion kann beispielsweise mit Hilfe des Bayer-Prozesses geschehen (DE-AS 1 095 808; DE-AS 1 131 658; Erdöl und Kohle/Erdgas/Petrochemie 16, 520 bis 523 [1963]). Diese sogenannte »Kalthydrierung« wird in einem separaten Reaktor in flüssiger Phase bei einer Temperatur von 10 bis 20°C über fest angeordneten Palladium-Katalysatoren in der Rieselphase ausgeführt. Bei einer $C_4$-Fraktion aus der pyrolytischen Crackung läßt sich so der Vinylacetylen-Gehalt von 1300 ppm auf etwa 50 ppm in Gegenwart von bis zu 30 Gew.-% Butadien herabsetzen.

Eine andere Möglichkeit, Acetylen-Verbindungen aus Olefinen zu entfernen, ist ihre Umsetzung zu höhersiedenden Verbindungen in Gegenwart von Kupfer-(I)- und Kupfer-(II)-Salzlösungen (Chemie Ing.-Techn. 34, 120 [1962]).

Die Isolierung des Butadiens aus einem Gemisch der $C_4$-Kohlenwasserstoffe ist durch einfache Destillation nicht möglich, da alle darin enthaltenen Komponenten in einem sehr engen Temperaturbereich sieden und teilweise Azeotrope bilden. Es existieren zwei in der chemischen Technik geläufige Butadien-Isolierverfahren, die auf einem chemischen und physikalischen Trenneffekt beruhen.

Ein von der Fa. Esso entwickeltes Extraktionsverfahren zur Aufarbeitung von $C_4$-Schnitten nutzt die Komplexbildung von Butadien mit Kupferammonacetat aus. Die eingesetzten $C_4$-Schnitte sollten zur Verminderung der acetylenischen Kohlenwasserstoffe vorhydriert sein, da sonst der Extraktionsprozeß durch Schaumbildung gestört wird. Die sich trotzdem anreichernden $C_4$-Acetylene in diesem Prozeß stellen eine laufende Detonationsgefahr dar. Eine aufwendige Regenerierung der Extraktionslösung wirkt sich auf das Verfahren in wirtschaftlicher Hinsicht negativ aus (Oil and Gas J. 55 [35], 113 [1957]).

Alle modernen Verfahren der Butadiengewinnung wenden das Prinzip der Extraktivdestillation mit selektiven organischen Lösungsmitteln an. Je nach Anwendung dieser verschiedenen Extraktionsverfahren ist eine vorherige Acetylen-Entfernung notwendig oder mindestens zweckmäßig. Die älteren Verfahren, die Aceton, Furfurol oder Acetonitril verwenden, benutzen eine partielle $C_4$-Alkinhydrierung, um eine störende Harzbildung zu vermeiden. Die neueren Verfahren setzen als Lösungsmittel N-Methyl-pyrrolidon (BASF) bzw. Dimethylformamid (Nippon Zeon) ein. Sie verarbeiten die heute anfallenden butadienreichen Roh-$C_4$-Schnitte mit relativ hohen Anteilen an Alkinen, wie beispielsweise Methyl-, Ethyl- und Vinylacetylen, in Teilschritten des Verfahrensablaufes. Der größte Butadienverlust wird durch den Butadiengehalt von etwa 40% in dem $C_4$-Acetylenstrom, der meist verbrannt wird, verursacht. Mit zunehmender Verschärfung der Butadienspezifikation und mit höher werdenden Gehalten an Acetylenen im Roh-$C_4$-Schnitt haben sich als die am besten geeigneten Verfahren die von BASF und Nippon Zeon herausgestellt. Bei dem BASF-Verfahren wird eine Extraktivdestillation in mehreren Kolonnen mit N-Methyl-pyrrolidon als Lösungsmittel durchgeführt. Dabei werden die Butene über Kopf der Hauptwäsche abgezogen, das Butadien mit Vinylacetylen und anderen ungesättigten störenden Komponenten in der Mitte der Destillationseinheit. Es folgt anschließend eine weitere aufwendige Extraktivdestillation mit N-Methyl-pyrrolidon zur Trennung von Butadien und den $C_4$-Acetylen-Verbindungen. Bei diesen $C_4$-Acetylen-Verbindungen in reiner bzw. hochkonzentrierter Form besteht unter erhöhtem Druck die Gefahr der Selbstzersetzung. Der Butadiengehalt kann deshalb im Abfallstrom nicht auf unter 40% abgesenkt werden, wenn man nicht das Risiko der Selbstzersetzung im Falle einer Betriebsstörung eingehen will.

Eine mögliche Aufeinanderfolge der beschriebenen Einzelverfahren im Zusammenhang mit der Behandlung von Destillationsschritten aus der thermischen oder katalytischen Crackung von Kohlenwasserstoffgemischen sei am Beispiel der $C_4$-Fraktion dargestellt.

2

Bei der Aufarbeitung von $C_4$-Fraktionen, wie sie beispielsweise bei Crack-Prozessen anfallen, wird im allgemeinen zunächst das vorhandene Butadien ganz oder weitgehend entfernt. Es hinterbleibt dann ein Gemisch, das im wesentlichen Buten-(1), Buten-(2), Isobuten und gesättigte $C_4$-Kohlenwasserstoffe enthält. Diese als Raffinat I bezeichneten Gemische werden im allgemeinen aufgearbeitet, indem man in solchen Gemischen das Isobuten zu technisch interessanten Produkten umsetzt und diese abtrennt. Das dann verbleibende, im wesentlichen Buten-(1), Buten-(2) und gesättigte $C_4$-Kohlenwasserstoffe enthaltende Gemisch, das als Raffinat II geläufig ist, kann beispielsweise zur Herstellung von reinem Buten-(1), einem hochwertigen Comonomeren bzw. Homomonomeren, verwendet werden oder weiter nach dem Umsatz mit Isobuten als Alkylatbenzin verwendet werden.

Die genannte Umsetzung von Isobuten zu technisch interessanten Folgeprodukten kann beispielsweise darin bestehen, daß man das Isobuten mit Alkanolen unter Bildung von Alkyl-tert.-butylethern umsetzt. Insbesondere ist hier die Veretherung mit Methanol unter Bildung von Methyl-tert.-butylether (MTBE) von Interesse. Als Katalysator für diese Veretherungsreaktion verwendet man im allgemeinen saure Kationenaustauscher. Die gebildeten Ether, insbesondere MTBE, werden von den verbleibenden $C_4$-Kohlenwasserstoffen und gegebenenfalls von überschüssigem Alkohol abgetrennt (Hydr. PROC. 1977 [11], 185; Hydr. PROC. 1977 [12], 98—102; Oil and Gas J. 1976 [1], 76—77).

MTBE und Ethyl-tert.-butylether werden als octanzahlerhöhende Zusätze zu Kraftstoffen für Ottomotoren verwendet (Hydr. PROC. 1980 [2], 57—59). Höhere Alkyl-tert.-butylether finden als Lösungs- und Verdünnungsmittel Verwendung [NL-Patentanmeldung 74/10872; DE-OS 2 620 011).

Es sind aber auch Verfahren bekannt, bei denen die Veretherung des i-Butens mit Alkanolen im Roh-$C_4$-Schnitt in Gegenwart von Butadien und in Gegenwart der $C_4$-Acetylen-Verbindungen durchgeführt wird. So beschreibt DE-OS 2 521 673 ein Verfahren, bei dem eine selektive Veretherung von i-Buten in Anwesenheit größerer Mengen Butadien so durchgeführt wird, daß der Verlust an Butadien weniger als 2% beträgt. Die Arbeitsbedingungen werden hierbei so gewählt (60 bis 120° C, liquid hourly space velocity 5 bis 35 bzw. 0,5 bis 25), daß die Sekundärreaktion kinetisch gesteuert werden können. Geschieht dies nicht, so treten Butadienverluste von 20 bis 30% auf.

US 4 161 496 beschreibt die Veretherung des i-Buten im Roh-$C_4$-Schnitt in Gegenwart von Butadien in zwei aufeinanderfolgenden Reaktoren, wobei im zweiten Reaktor ein mit Natriumchlorid-Lösung abgestumpfter saurer Kationenaustauscher eingesetzt wird.

Die DE-AS 2 521 963 beschreibt ein Verfahren in zwei Stufen, wobei zur Erzielung einer hohen Selektivität und Ausbeute an MTBE in der ersten Stufe Methanol im Überschuß und in der zweiten Stufe i-Buten im Überschuß und somit insgesamt stöchiometrisch gleichwertig gearbeitet wird. Dabei wird in einer LHSV (liquid hourly space velocity) von 20 bis 50 gearbeitet, um Nebenreaktionen des i-Butens zu vermeiden.

Eine technisch interessante Umsetzung des i-Butens besteht darin, daß man das i-Buten an sauren Kontakten, beispielsweise in Gegenwart von Mineralsäuren oder sauren Kationenaustauschern zu Dimeren, Trimeren und höheren Oligomeren umsetzt und diese Oligomeren abtrennt. Diese Umsetzung wird vielfach in Schlaufenreaktoren durchgeführt (Erdöl und Kohle/Erdgas/Petrochemie 19 [7], 497 bis 500 [1966]). Diese Oligomeren können Ausgangsprodukte für die Oxosynthese sein, beispielsweise als Grundlage für Weichmacherprodukte (Erdöl und Kohle 27 [2], 77 bis 82 S[1974]) oder finden, auch in hydrierter Form, als Oktanzahl-erhöhende Zusätze zu Kraftstoffen (Hydr. PROC. 1973 [4], 171 bis 173) und als aromatenfreie Lösungs- und Verdünnungsmittel großtechnische Anwendung.

Für alle Umsetzungen des im Raffinat II enthaltenen Buten-(1) ist es vorteilhaft, wenn nach der Umsetzung des i-Butens und der Abtrennung der daraus hervorgehenden Reaktionsprodukte dieses Raffinat II einen niedrigen Anteil an diolefinischen, acetylenischen und sauerstoffhaltigen Verbindungen aufweist, da diese Verbindungen bei den genannten Weiterverarbeitungen eine Katalysatordesaktivierung verursachen oder Polymerisate und schwer abtrennbare Azeotrope bilden.

Diese beispielhaft für den $C_4$-Schnitt angegebenen Einzelreaktionen und zusammenhängenden Reaktionsfolgen können in analoger Weise für einen $C_5$-Schnitt oder für andere Destillationsschnitte, beispielsweise im $C_3$—$C_8$-Bereich, beschrieben werden.

Bisher ist kein Verfahren bekanntgeworden, gemäß dem man bei der Umsetzung von tertiären Olefinen gleichzeitig die acetylenischen und diolefinischen Kohlenwasserstoffe sowie die Carbonyl-Verbindungen entfernen kann. Hierzu sind bisher separate Verfahrensschritte notwendig.

Es wurde nun ein Verfahren zur katalytischen Umsetzung von i-Alkenen und zur weitgehenden Entfernung von Acetylen-Verbindungen und gegebenenfalls von Diolefinen durch katalytische Hydrierung in Kohlenwasserstoffströmen, die i-Alkene, Acetylen-Verbindungen und gegebenenfalls Diolefine enthalten, gefunden, das dadurch gekennzeichnet ist, daß man die katalytische Umsetzung und die katalytische Hydrierung gleichzeitig an einem makroporösen oder gelförmigen Kationenaustauscher in der H$^+$-Form, der 0,001 bis 10 g eines oder mehrerer Metalle der VI. und/oder VII. und/oder der VIII. Nebengruppe des Periodensystems der Elemente in elementarer Form pro Liter trockenen Kationenaustauschers enthält und einen Vernetzungsgrad von 2 bis 65% sowie eine spezifische Oberfläche von 5 bis 750 m²/g trockenes Austauscherharz aufweist, in flüssiger Phase bei 20 bis 140° C durchführt.

Ein wesentliches Merkmal des erfindungsgemäßen Verfahrens ist die Umsetzung in Gegenwart eines makroporösen oder gelförmigen sauren Kationenaustauschers in der H$^+$-Form, der 0,001 bis 10 g eines oder mehrerer Metalle der VI. und/oder VII. und/oder VIII. Nebengruppe des Periodensystems der Elemente in elementarer Form pro Liter trockenen Kationenaustauschers enthält, wobei der Kationenaustauscher einen Vernetzungsgrad von 2 bis 65% und eine spezifische Oberfläche von 5 bis 750 m²/g trockenes Austauscherharz aufweist.

Erfindungsgemäß einzusetzende Kationenaustauscher sind beispielsweise durch Copolymerisation von Vinylmonomeren und Divinylvernetzern in Gegenwart von Lösungsmitteln herstellbar, wobei gegebenenfalls die Lösungsmittel die Monomeren zu lösen vermögen, aber keine Quellwirkung für die entstehenden Polymeren zeigen (DE-AS 1 113 570, US 3 586 646). Als Vinylmonomere können beispielsweise Styrol oder Acrylsäureester eingesetzt werden. Als Divinylvernetzer kommt beispielsweise Divinylbenzol in Frage. Der Vernetzungsgrad ist abhängig von der Menge des Divinylvernetzers und liegt im Bereich von 2 bis 65%, vorzugsweise von 8 bis 25%. Als Vernetzungsgrad wird hier die Menge des Divinylvernetzers, bezogen auf die Gesamtmenge an Comonomeren, verstanden.

Jedoch sind auch andere Kationenaustauscherharze, beispielsweise auf der Basis Phenol-Formaldehyd, erfindungsgemäß einsetzbar.

Als saure Gruppen können solche Kationenaustauscher beispielsweise Carboxylgruppen, Phosphonsäuregruppen oder Sulfonsäuregruppen enthalten. Carboxylgruppen +önnen beispielsweise durch Verseifung von Acrylester-Gruppen, Sulfonsäuregruppen beispielsweise durch nachträgliche Sulfonierung eingeführt werden.

Bevorzugt werden stark saure, Sulfonsäuregruppen enthaltende Styrol-Divinylbenzol-Polymerisate eingesetzt. Solche Kationenaustauscher sind unter verschiedenen Bezeichnungen im Handel erhältlich.

Vorzugsweise weisen die erfindungsgemäß einzusetzenden Kationenaustauscher eine spezifische Oberfläche von 50 bis 250 m²/g auf. Der mittlere Porenradius der Kationenaustauscher kann beispielsweise in Grenzen von 50 bis 1200 Å, vorzugsweise 70 bis 500 Å, variieren.

Wenn die Kationenaustauscher als Perlpolymerisate eingesetzt werden, können sie beispielsweise Korngrößen von 0,1 bis 2 mm aufweisen. Wenn sie als Pulverharz zum Einsatz gelangen, können sie beispielsweise Korngrößen von 10 bis 100 µm aufweisen.

In das erfindungsgemäße Verfahren werden die Kationenaustauscher in der $H^+$-Form eingesetzt, nachdem sie mit 0,001 bis 10 g, bezogen auf 1 l trockenen Kationenaustauscher, eines oder mehrerer Metalle der VI. und/oder VII. und/oder VIII. Nebengruppe des Periodensystems der Elemente, wie beispielsweise im CRC-Handbook of Chemistry and Physics, 60. Auflage, 1979/1980, herausgegeben von R. C. Weast, CRC-Press, Bota Raton, Florida, USA, enthalten, in elementarer Form belegt worden sind. Als Metalle der genannten Nebengruppen des Periodensystems kommen beispielsweise in Frage: Chrom, Molybdän, Wolfram, Mangan, Rhenium, Eisen, Kobalt, Nickel, Ruthenium, Rhodium, Palladium, Osmium, Iridium und/oder Platin. Bevorzugt seien genannt: Palladium, Platin, Rhenium, Molybdän oder Nickel. Ganz besonders bevorzugt sind Palladium, Platin und Nickel. Diese Metalle können einzeln oder als Gemisch mehrerer von ihnen auf dem Kationenaustauscher angebracht sein. Bevorzugt ist der Einsatz von jeweils nur einem der genannten Metalle. Für den Fall der Verwendung von Edelmetallen aus den genannten Gruppen, beispielsweise Palladium oder Platin, kann dessen Menge im unteren Teil des allgemeinen Bereichs gewählt werden; für den Fall der Verwendung von Nicht-Edelmetallen kommt der obere Teil des allgemeinen Bereichs in Frage. Vorzugsweise ist der Kationenaustauscher mit 0,2 bis 3 g Platin oder Palladium oder mit 1 bis 10 g Nickel pro Liter trockenen Austauschers belegt.

Die Belegung der Kationenaustauscher mit dem oder den Metallen kann beispielsweise so erfolgen, daß man zunächst das oder die gewünschten Metalle in Form einer Lösung, beispielsweise in Wasser, von nicht-komplexen, kationischen Salzen ansatzweise mit dem Kationenaustauscher in der $H^+$-Form in an sich bekannter Weise zusammenbringt. Solche Metallsalze können beispielsweise die Chloride, Bromide, Nitrate, Sulfate und/oder Acetate sein.

Die Menge des Salzes wird so gewählt, daß sich nach der weiteren Behandlung, insbesondere nach der Reduzierung, die gewünschte Menge Metall auf dem Kationenaustauscher befindet. Die einzusetzende Salzmenge kann gegebenenfalls durch einfaches Probieren ermittelt werden.

Es kann vorteilhaft sein, die beim Zusammenbringen des oder der Metallsalze mit dem Kationenaustauscher frei werdende Säure zu neutralisieren. Diese Neutralisation kann während oder nach dem Zusammenbringen des oder der Metallsalze mit dem Kationenaustauscher erfolgen. Für diese Neutralisation sind alkalisch reagierende Verbindungen, gegebenenfalls in Form einer wäßrigen Lösung, geeignet, wie Natriumhydroxid, Kaliumhydroxid, Ammoniak, Natriumcarbonat, Kaliumcarbonat und/oder Ammoniumcarbonat. Nach dem Zusammenbringen von Metallsalz(en) und Kationenaustauscher und gegebenenfalls nach der Neutralisation wird der Kationenaustauscher im allgemeinen neutral gewaschen und anschließend getrocknet, beispielsweise bei erhöhter Temperatur und gegebenenfalls bei vermindertem Druck. Geeignete Trocknungsbedingungen sind beispielsweise 80 bis 100°C und Wasserstrahlpumpenvakuum während etwa 24 Stunden.

Zur Überführung der aufgebrachten Metalle in den elementaren Zustand kann der so vorbehandelte Kationenaustauscher mit Wasserstoff behandelt werden. Diese Behandlung kann beispielsweise bei 2 bis 50 bar, vorzugsweise bei 20 bis 30 bar, und bei einer Temperatur im Bereich von etwa 50 bis 140°C, vorzugsweise 80 bis 120°C, durchgeführt werden.

Der erfindungsgemäß einzusetzende Kationenaustauscher kann bei diskontinuierlicher Arbeitsweise beispielsweise in Mengen von 1 bis 50 Gew.-Teilen Reaktionsgemisch, bezogen auf 1 Gew.-Teil Kationenaustauscher, eingesetzt werden. Bevorzugt werden 5 bis 50, besonders bevorzugt 10 bis 30 Gew.-Teile Reaktionsgemisch, bezogen auf 1 Gew.-Teil Kationenaustauscher. Bei kontinuierlicher Arbeitsweise kann der Kationenaustauscher beispielsweise in solchen Mengen eingesetzt werden, daß sich eine Belastung a = 0,1 bis 100, vorzugsweise 0,5 bis 20, besonders bevorzugt 1 bis 10, ergibt. a bedeutet dabei kg Kohlenwasserstoffe im Einsatz/kg Kationenaustauscher pro Stunde.

Die in das erfindungsgemäße Verfahren einsetzbaren Gemische mit den genannten Bestandteilen können von verschiedenster Herkunft sein, ohne daß dies die Durchführbarkeit des erfindungsgemäßen Verfahrens beeinflußt. Beispielsweise seien solche Stoffgemische genannt, die tertiäre $C_4$- bis $C_9$-Olefine und $C_3$—$C_9$-Kohlenwasserstoffe enthalten, die in Raffinerien oder petrochemischen Anlagen anfallen, beispielsweise nach der thermischen oder katalytischen Crackung von Gasöl, Naphtha, LNG (liquid natural gas) oder Butanen anfallen. Hierbei können diese Stoffströme die rohen Stoffströme aus der Crackung oder die verschiedensten Raffinate sein, die nach dem weitgehenden Entzug von gewünschten Wertstoffen, wie Diolefinen oder tert.-Olefinen entstehen. Alle Stoffströme sind jedoch durch einen Gehalt an i-Alkenen, Acetylen-Verbindungen und Diolefinen ausgezeichnet. Als i-Alkene seinen tertiäre olefinische Kohlenwasserstoffe mit 4—9, wie i-Buten, i-Amylen, i-Nonen, bevorzugt mit 4—6 C-Atomen genannt. Besonders bevorzugt ist i-Buten. Als Acetylene seien $C_3$—$C_5$-Alkine genannt, wie Methylacetylen, Ethylacetylen, 1,2-Dimethylacetylen, Vinylacetylen und Propylacetylen. Als Diolefine seien solche kmit 4—6, bevorzugt 4—5 C-Atomen genannt, wie Butadien-1,2, Butadien-1,3, Pentadien-1,3 und Isopren. Diese genannten Verbindungen sind zumindest in Restmengen in den erfindungsgemäß einzusetzenden Stoffströmen vorhanden. Für den Fall, daß als Diolefin Butadien vorliegt, sei für dieses ein Gehalt von 0,001—70 Vol.-%, bezogen auf das Volumen des Gesamtstroms, genannt. Die einsetzbaren Stoffgemische enthalten ferner Monoolefine und Alkane mit 3—9, bevorzugt 3—6 C-Atomen. In besonders bevorzugter Weise haben in den eingesetzten Gemischen alle genannten Stoffe überwiegend die gleiche Anzahl von C-Atomen mit nur untergeordneten Anteilen an Stoffen mit den jeweils benachbarten Zahlen an C-Atomen, wie sie in Raffinerien und petrochemischen Anlagen anfallen. Die vorgenannten Stoffgemische können ferner Carbonyl-Verbindungen, also Aldehyde und/oder Ketone enthalten, wie Aceton oder Methylethylketon.

Am Beispiel von $C_4$-Stoffströmen aus der Crackung seien typische Gehalte bzw. Zusammensetzungen genannt:

a) Gehalte an Stoffen im Roh-$C_4$-Crackerschnitt in Gew.-%.

| | |
|---|---|
| $C_3$-KW | 0,3—0,16 |
| Butadien-(1,3) | 26—70 |
| i-Buten | 20—9 |
| Methylacetylen | 0,06—0,1 |
| Vinylacetylen | 0,15—3,0 |
| Ethylacetylen | 0,04—0,55 |
| Carbonyle [ppm] | 150—200 |
| n-Butan, i-Butan | 3—6 |
| n-Butan-(1), -(2) | 20—30 |

b) $C_4$-Raffinat I nach weitgehender Entfernung des Butadiens, getrennt nach seiner Herkunft aus einem thermischen oder katalytischen Crackverfahren.

| | Aus Steam-Cracker Gew.-% | Aus Cat-Cracker Gew.-% |
|---|---|---|
| n-Butan | 6—8 | ca. 10 |
| i-Butan | 2—3 | ca. 34 |
| i-Buten | 40—50 | 15—22 |
| Buten-(1) | 24—28 | ca. 13 |
| Buten-(2) | 19—21 | ca. 28 |
| Butadien | ca. 0,15 | ca. 0,5 |
| Acetylene | 0,01 | ca. 0,01 |
| Carbonyle | 0,02 | 0,02 |

5

Völlig überraschend bei dem neuen Verfahren ist die Tatsache, daß sich die Acetylen-Verbindungen weitgehend hydrieren lassen, während die katalytische Umsetzung der i-Alkene bis zu kleinsten Gehalten an i-Alken weiterläuft. Als weiterhin sehr vorteilhaft erweist es sich, daß erfindungsgemäß die Acetylen-Verbindungen unter dem Einfluß wechselnder Wasserstoffmengen in ihrem Gehalt wahlweise vermindert werden können, ohne die Diolefine, beispielsweise das Butadien, wesentlich anzugreifen. Der gewünschte Umwandlungsgrad durch Hydrierung kann beispielsweise durch die Kontaktbelastung, die Wasserstoffmenge bzw. den Wasserstoffdruck oder die Temperatur eingestellt werden. Durch einfaches Ausprobieren kann für verschieden zusammengesetzte Gemische die Summe der günstigsten Bedingungen gefunden werden. Bei nur noch geringen Gehalten an Diolefinen, beispielsweise an Butadien im Raffinat I oder im Raffinat II, können jedoch auch diese Diolefine durch Hydrierung weitgehend entfernt werden. Bei einem Gehalt an carbonyl-Verbindungen werden diese in den meisen Fällen ebenfalls weitgehend entfernt.

Als Wasserstoffmenge sei beispielsweise 0,001 bis 1 Mol Wasserstoff, bevorzugt 0,02 bis 0,5 Mol, besonders bevorzugt 0,05 bis 0,2 Mol, pro Mol des umzusetzenden Eingangsgemisches genannt. Als Molzahl für das Eingangsgemisch sei hierbei die Misch-Molzahl aller im Eingangsstrom enthaltenen Stoffe verstanden.

Der in das erfindungsgemäße Verfahren einsetzbare Wasserstoff kann sowohl reiner Wasserstoff, technisch reiner Wasserstoff mit den üblichen Verunreinigungen oder ein stärker verunreinigter, technischer roher Wasserstoff sein. Die Wasserstoffmenge in einem solchen Wasserstoff enthaltenden Gas beträgt beispielsweise 40 bis 100 Vol.-%, bevorzugt 85 bis 99 Vol.-%, besonders bevorzugt über 95 Vol.-%. Bezogen auf den Kontakt sei eine zugeführte Wasserstoffmenge von 0,001 bis 100, bevorzugt 0,01 bis 50, besonders bevorzugt 0,1 bis 20 l Wasserstoff pro l Kontakt genannt.

Als katalytische Umsetzung der i-Alkene sei beispielsweise ihre Veretherung mit niederen Alkanolen und/oder ihre katalytische Oligomerisierung zu Dimeren, Trimeren, Tetrameren oder zu höheren Oligomeren genannt.

Für den Fall, daß als katalytische Umsetzung der i-Alkene ihre Veretherung verstanden wird, sei als niederer Alkohol ein solcher mit 1 bis 8, bevorzugt 1 bis 4, besonders bevorzugt 1 oder 2 C-Atomen genannt, wie Methanol, Ethanol, Propanol, Butanol, Hexanol, Octanol. In ganz besonders bevorzugter Weise wird mit Methanol verethert. Das Molverhältnis von Alkanol zu dem i-Alken beträgt beispielsweise 0,1—4 : 1, bevorzugt 0,8—1,2 : 1, besonders bevorzugt 0,95—1,1 : 1.

Das Alkanol und das i-Alken werden hierbei zu dem zugehörigen Alkyl-tert.-isoalkylether umgesetzt, beispielsweise entsteht aus Methanol und i-Buten Methyl-tert.-butylether (MTBE).

Bei der katalytischen Umsetzung der i-Alkene in Gegenwart eines Alkanols wird der unerwünschte Umsatz der linearen Monoolefine sehr stark zurückgedrängt.

Das erfindungsgemäße Verfahren wird in flüssiger Phase bei einer Temperatur von 20 bis 140° C, bevorzugt 20 bis 120° C, besonders bevorzugt 25 bis 100° C, und in ganz besonders bevorzugter Weise 30 bis 80° C, durchgeführt. Der Druck wird so gewählt, daß bei gegebener Temperatur zumindest ein Teil der Ausgangsstoffe in flüssiger Phase vorliegt. Dies kann im allgemeinen erreicht werden, wenn man Drücke im Bereich von 2 bis 100 bar, bevorzugt 3 bis 30 bar, anwendet. Nach Beendigung der Reaktion kann man das Reaktionsgemisch in an sich bekannter Weise aufarbeiten. Vorzugsweise trennt man im Falle einer diskontinuierlichen Arbeitsweise zunächst den Kationenaustauscher ab, beispielsweise durch Dekantieren und Zentrifugieren. Der Kationenaustauscher kann dann mindestens 20mal wieder verwendet werden, ohne eine nennenswerte Abnahme der Aktivität zu zeigen. Aus dem entnommenen Reaktionsprodukt kann man für den Fall, daß in Gegenwart eines Alkanols gearbeitet wurde, den gebildeten Alkyl-tert.-alkylether und gegebenenfalls vorhandenes nicht-umgesetztes Alkanol abtrennen, beispielsweise durch Destillation. Das entnommene Reaktionsprodukt kann beispielsweise nach Durchlaufen eines Debutanizers als Vergaserkraftstoff verwendet werden. Sofern das Reaktionsprodukt nicht-umgesetztes Alkanol enthält, ist es bevorzugt, dieses Alkanol abzutrennen und erneut in das erfindungsgemäße Verfahren einzusetzen. In einer diskontinuerlichen Variante kann das erfindungsgemäße Verfahren unter Zusatz von Alkanolen beispielsweise wie folgt durchgeführt werden:

In einem geschlossenen Reaktor wird das zu verethernde i-Alken, das sich im Gemisch mit einem oder mehreren weiteren Mono- oder Diolefinen, Acetylen-Verbindungen und Carbonyl-Verbindungen und/oder weiteren inerten Kohlenwasserstoffen befindet, mit dem Alkanol im vorgewählten molaren Verhältnis und mit gasförmigem Wasserstoff in Gegenwart eines der oben beschriebenen Kationenaustauscher im Verhältnis von beispielsweise 10 bis 30 Gew.-Teilen Reaktionsgemisch pro Gew.-Teil Kationenaustauscher versetzt und etwa 0,1 bis 5 Stunden unter dem o. g. Druck und bei der o. g. Temperatur gerührt oder geschüttelt, bis die Veretherung und Umsetzung der Acetylen- und Carbonyl-Verbindungen abgeschlossen ist. Danach wird das Reaktionsgemisch entspannt und der Kationenaustauscher abgetrennt.

In einer kontinuierlichen Variante des erfindungsgemäßen Verfahrens kann unter Zusatz von Alkanolen beispielsweise wie folgt verfahren werden:

Einer der oben beschriebenen Kationenaustauscher wird in einen temperierbaren Reaktor, beispielsweise in ein oder mehrere temperierbare Reaktionsrohre, eingefüllt. Das oben beschriebene, das i-Alken enthaltende Gemisch, Wasserstoff und das Alkanol werden einzeln oder als Gemisch von

6

unten nach oben oder von oben nach unten, beispielsweise in der Rieselphase, durch das Austauscherbrett geführt. Vorzugsweise arbeitet man hier mit einer konstanten Fließgeschwindigkeit. Der den Reaktor verlassende Produktstrom wird gesammelt, und der Gehalt an nicht-umgesetzten Acetylen-Kohlenwasserstoffen und Carbonyl-Verbindungen und Ethern kann darin beispielsweise durch gaschromatographische Analyse bestimmt werden. Die erhaltenen Produkte können, wie zuvor beschrieben, aufgearbeitet und verwendet werden.

Als katalytische Umsetzung kommt weiterhin die Oligomerisierung der i-Alkene in Frage, beispielsweise die Dimerisierung, Trimerisierung usw. Wird die katalytische Umsetzung in Gegenwart eines Alkanols durchgeführt, findet neben der Oligomerisierung immer eine Veretherung statt. Bei höheren Mengen Alkanol, insbesondere bei mindestens stöchiometrischen Mengen Alkanol, wird die Oligomerisierung weitgehend zurückgedrängt.

Die Veretherung als katalytische Umsetzung hat Bedeutung für Roh-Crackschnitte und für Raffinate im Anschluß an die Entfernung der Diolefine, beispielsweise für Roh-$C_4$-Schnitte und $C_4$-Raffinate I. Die Oligomerisierung als katalytische Umsetzung hat Bedeutung besonders für Raffinate nach der Entfernung der Diolefine, insbesondere für $C_4$-Raffinat I und $C_4$-Raffinat II.

Das erfindungsgemäße Verfahren weist eine Reihe von überraschenden Vorteilen auf, von denen die wichtigsten im folgenden aufgezählt sind:

1. Für den Fall, daß unter Zusatz eines Alkanols gearbeitet wird, lassen sich in i-Alkene enthaltenden Kohlenwasserstoffmischungen auch in Gegenwart großer Mengen Butadien die in den nachfolgenden Trennoperationen und Prozessen störenden Acetylen-Verbindungen und Carbonyl-Verbindungen bei gleichzeitiger Herstellung des entsprechenden Alkyl-tert.-alkylethers entfernen. Der Grad dieser Entfernung läßt sich mengenmäßig den jeweiligen Bedürfnissen anpassen.
2. Damit ergibt sich nach der Entfernung der Acetylen-Verbindungen und gegebenenfalls der Carbonyl-Verbindungen sowie der Umsetzung des i-Alkens mit einem Alkanol für die nachfolgende Butadien-Abtrennung der Wegfall eines technisch und energetisch aufwendigen Verfahrensteils der verschiedenartigen Butadien-Extraktionsverfahren.
3. Die Entfernung der Acetylen- und gegebenenfalls der Carbonyl-Verbindungen vermeidet störende Harzbildungen in den Butadien-Extraktionsanlagen, gewährleistet längere Laufzeiten der Produktionsanlagen und spart kostenintensive Reinigungsoperationen der Kolonnen und Apparate.
4. Eine besondere Vorhydrierung von Roh-$C_4$-Schnitten in extra dafür einzusetzenden Hydrierreaktoren entfällt.
5. Ebenso entfällt eine besondere Vorbehandlung zur Entfernung von stärker ungesättigten Verbindungen und gegebenenfalls Carbonylverbindungen aus dem Raffinat II vor dessen Einsatz in eine Buten-(1)-Gewinnung, in Alkylierungen mit Buten-(1) oder dessen Verwendung als Buten-(1)-Comonomer mit Ethylen zu LLDPE (linear low density polyethylene).
6. Die erfindungsgemäß eingesetzten metallbeladenen Kationenaustauscher bewirken aufgrund der gleichmäßigen Metallverteilung eine gute Wärmeabfuhr und eine hohe Selektivität in ihrer katalytischen Wirkung. Dadurch kommt es im Kationenaustauscherbett zu keinen nennenswerten Temperaturspitzen, die zu Kontaktschädigungen führen können. Weiterhin kann das erfindungsgemäße Verfahren bei relativ niedrigen Temperaturen durchgeführt werden. Die relativ niedrige Reaktionstemperatur macht das Anfahren und Abfahren der Reaktoren problemlos.
7. Bereits in der chemischen Technik in Betrieb befindliche Veretherungsreaktoren können nach Einbau einer Wasserstoffzudosierung und Befüllung mit metalldotierten Kationenaustauschern auf die Vorteile des erfindungsgemäßen Verfahrens leicht umgerüstet werden.

Im erfindungsgemäßen Verfahren werden beispielsweise in einem $C_4$-Schnitt nach der Abtrennung der Umsetzungsprodukte der katalytischen Umsetzung des i-Butens (zu den Ethern oder zu den Oligomeren) und gegebenenfalls nach der Abtrennung des Butadien Kohlenwasserstoffströme erhalten, die weitgehend von i-Buten und den Acetylen- und Carbonyl-Verbindungen befreit sind. Hierbei wird die Umsetzung von Buten-(1) zu Buten-(2), zu ihren Copolymeren oder zu Butanen weitgehend zurückgedrängt. Dieses Kohlenwasserstoff-Gemisch hat im allgemeinen einen Gehalt an i-Alken von 0,1 bis 1 Gew.-%, von Butadien von weniger als 100 ppm, an Acetylen-Verbindungen von kleiner als 50 ppm und von Carbonyl-Verbindungen unter 10 ppm.

Ein solches Kohlenwasserstoffgemisch kann ohne weitere Reinigung in die Buten-(1)-Gewinnung, in Alkylierungsreaktionen mit dem Buten-(1) oder in die Oligomerisierung dieses Buten-(1) eingesetzt werden.


Beispiel 1
(Herstellung eines erfindungsgemäß zu verwendenden Kationenaustauschers)

Entsprechend der DE-AS 1 113 570, Beispiel 3, wurde ein makroporöser Kationenaustauscher hergestellt. Der wasserfeuchten $H^+$-Form dieses Kationenaustauschers wurde im Batch so viel Palladiumacetat angeboten, daß 1 g Palladium pro Liter trockenes Harz nach Reduktion mit Wasserstoff auf dem Kationenaustauscher vorhanden waren. Die bei der Behandlung mit Palladiumacetat freigesetzte Säure wurde mit 1gew.-%iger Natronlauge neutralisiert. Anschließend wurde der neutral gewaschene Kationenaustauscher 24 Stunden bei 100°C im Vakuum einer Wasserstrahlpumpe getrocknet. Das auf dem Kationenaustauscher befindliche Palladium wurde dann zwischen 90 und 100°C mit Wasserstoff bei einem Druck von 20 bis 25 bar innerhalb von 48 Stunden zum Metall reduziert.

## Beispiel 2

Als Hydrier- und Veretherungsapparatur fand ein temperierbarer Durchlaufreaktor mit einem lichten Reaktordurchmesser von 20 mm Anwendung. Es wurde so viel des gemäß Beispiel 1 hergestellten Kationenaustauschers eingefüllt, daß sich in Relation zu der eingesetzten Flüssigkeitsmenge eine Belastung des Kationenaustauschers von a = 5,6 ergab. Der Wert a bedeutet hierbei kg Einsatz-Kohlenwasserstoffgemisch pro kg Kationenaustauscher pro Stunde. Zur Temperaturkontrolle ist der Reaktor mit mehreren Temperaturmeßstellen in Abständen von jeweils 100 mm ausgerüstet. Die Reaktionstemperatur wurde bei 60°C gehalten. Der Reaktor war über eine Druckhaltung druckgeregelt. Der Druck wurde auf 8 bar gehalten. Das eingesetzte Kohlenwasserstoffgemisch wurde mit Methanol in einer Mischkammer vor dem Reaktor vermischt. Gasförmiger reiner Wasserstoff wurde dem Reaktor mengengeregelt separat eingegeben. Das den Reaktor verlassende Reaktionsprodukt wurde in einem gekühlten Abscheider zwischengelagert, bevor es anschließend destillativ aufgearbeitet werden konnte. Dabei wurde nach der Wasserstoffabtrennung in einer Destillationskolonne am Kopf ein isobutenarmes, alkinarmes $C_4$-Kohlenwasserstoffgemisch und am Sumpf Methyl-tert.-butylether entnommen.

Aus diesem Sumpfprodukt wurde in einer nachgeschalteten Destillation reinster Methyl-tert.-butylether gewonnen.

Die Analysen sind gaschromatographisch ausgeführt worden. Die Probenentnahme zur gaschromatographischen Bestimmung der Endprodukte erfolgte nach einer Reaktionszeit von 6 Stunden bei konstanter Kationenaustauscherbelastung. Die Zusammensetzung des Einsatz- und Endproduktes und sonstige Kenndaten der Reaktion sind aus Tabelle I ersichtlich.

## Beispiel 3 (Vergleichsbeispiel)

Entsprechend der DE-PS 1 113 570, Beispiel 3, wurde ein makroporöser Kationenaustauscher hergestellt. Das so erhaltene wasserfeuchte Ionenaustauscherharz wurde zur Entfernung des Wassers im Trockenschrank bei 80 bis 100°C und 15 Torr über Nacht getrocknet. Der so erhaltene trockene Kationenaustauscher, der kein Metall der VI., VII. und/oder VIII. Nebengruppe des Periodensystems enthielt, wurde in eine Beispiel 2 entsprechende Umsetzung eingesetzt.

Die Zusammensetzung des Einsatz- und Endproduktes und sonstige Kenndaten der Reaktion sind aus Tabelle 1 ersichtlich.

## Beispiel 4 und 5

Die Versuche wurden, wie in Beispiel 2 beschrieben, durchgeführt, jedoch wurde der handelsübliche Bayer-Kationenaustauscher LEWATIT SPC 118 (Styrol-Divinyl-Copolymer mit Sulfonsäuregruppen), nach Beispiel 1 mit Palladium dotiert, eingesetzt. Die zugegebene Wasserstoffmenge ist jeweils auf ca. 15 bzw. 18 l/h erhöht. Als Ergebnis dargestellt in Tabelle 1 nimmt der $C_3$- und $C_4$-Acetylen-Umsatz von 95,5% in Beispiel 2 auf 96,6% bzw. 99% in den angeführten Beispielen 4 und 5 zu. Mit zunehmendem $C_3$-, $C_4$-Acetylen-Umsatz nimmt der Butadienverlust zu, der durch die Buten-(1)-Zunahme wieder kompensiert wird.

## Beispiel 6

Der Versuch wurde, wie in Beispiel 4 beschrieben, jedoch mit einem butadienarmen $C_4$-Schnitt, sogenanntem Raffinat durchgeführt. Reaktionsbedingungen, die Zusammensetzung der Einsatz- und Endprodukte der Umsetzung sind aus Tabelle 2 ersichtlich. Es tritt die beabsichtigte Isobutenveretherung und Verminderung des Butadiengehaltes ein.

## Beispiel 7 (Vergleichsbeispiel)

Der Versuch wurde, wie in Beispiel 3 beschrieben, durchgeführt, jedoch mit dem Einsatzprodukt wie in Beispiel 6. Aus Tabelle 2 sind alle Versuchsparameter und Stoffzusammensetzungen ersichtlich.

Tabelle 1

Roh-$C_4$-Veretherung in Gegenwart von Wasserstoff an metalldotierten Kationenaustauschern

| Kontaktmenge: | 120 ml ≙ 55,2 g |
| Versuchsbedingungen: | p = 8 bar; T = 60° C; WHSV = 5,6 |

| Zusammensetzung | Einsatz | Beispiel 2 KAT H+/Pd | 3 (Vergleich) KAT H+-Form | 4 SPC 118 H+/Pd | 5 SPC 118 H+/Pd |
|---|---|---|---|---|---|
| i-Buten (Gew.-%) | 19,0 | 3,6 | 1,8 | 2,8 | 4,2 |
| Buten-(1) (Gew.-%) | 12,2 | 13,7 | 12,4 | 15,8 | 16,5 |
| cis-/tr.-Buten-(2) (Gew.-%) | 7,5 | 8,1 | 7,4 | 9,5 | 10,3 |
| Butadien-(1,2) (Gew.-%) | 0,29 | 0,2 | 0,3 | 0,2 | 0,1 |
| Butadien-(1,3) (Gew.-%) | 37,8 | 36,6 | 37,4 | 33,0 | 31,6 |
| $\sum C_4$-Acetylene (Gew.-%) | 0,89 | 0,04 | 0,75 | 0,02 | 0,01 |
| Carbonyle (ppm) | 180 | 20 | 160 | 20 | 20 |
| Methanol (Gew.-%) | 12,7 | 3,9 | 2,9 | 3,5 | 4,2 |
| MTBE (Gew.-%) | 0,1 | 24,2 | 27,1 | 25,7 | 23,3 |
| Umsatz: i-Buten (%) | — | 81,0 | 90,5 | 85,3 | 77,9 |
| Butadien-Ausb. (%) | — | 96,8 | 98,9 | 87,3 | 83,6 |
| Buten-(1)-Zunahme (%) | — | 12,3 | 1,6 | 29,5 | 35,2 |
| Buten-(2)-Zunahme (%) | — | 8,0 | — | 26,7 | 37,3 |
| Carbonyl-Umsatz (%) | — | ca. 90 | 11,1 | ca. 90 | ca. 90 |
| $\sum C_4$-Acetylen-Umsatz | — | 95,5 | 15,7 | 96,6 | 99 |
| Wasserstoffzugabe [l · h$^{-1}$] | | ca. 6 | ca. 6 | ca. 15 | ca. 18 |

9

Tabelle 2

Raffinat-I-Veretherung in Gegenwart von Wasserstoff an metalldotierten Kationenaustauschern

Kontaktmenge: 120 ml $\hat{=}$ 55,8 g
Versuchsbedingungen: $p = 8$ bar; $T = 60°C$; WHSV $= 5,6$; $H_2 = 1,2\, l \cdot h^{-1}$

| Zusammensetzung | Einsatz | Beispiel 6<br>SPC 118<br>$H^+/Pd$ | 7<br>(Vergleich)<br>SPC 118 |
|---|---|---|---|
| Butane (Gew.-%) | 14,0 | 15,1 | 16,6 |
| i-Buten (Gew.-%) | 32,5 | 4,2 | 4,3 |
| Buten-(1) (Gew.-%) | 22,7 | 14,9 | 21,0 |
| cis-/tr.-Buten-(2) (Gew.-%) | 13,9 | 20,9 | 13,6 |
| Butadien-(1,3) (Gew.-%) | 0,23 | 0,05 | 0,23 |
| Carbonyl-Verb. (ppm) | 200 | 20 | 180 |
| Acetylen-Verb. (ppm) | 10 | <10 | 10 |
| Methanol (Gew.-%) | 16,6 | 0,4 | 0,5 |
| MTBE (Gew.-%) | 0,1 | 44,5 | 44,3 |
| Umsatz: i-Buten (%) | — | 87,1 | 86,7 |
| Umsatz: Butadien-(1,3) (%) | — | 78,3 | — |
| Umsatz: Carbonyle (%) | — | >90 | 10 |

### Beispiel 8

Der Versuch wurde, wie in Beispiel 6 beschrieben, jedoch mit dem Unterschied durchgeführt, daß in Abwesenheit von Methanol mit Wasserstoff umgesetzt wurde.

Reaktionsbedingungen, die Zusammensetzung der Einsatz- und Endprodukte der Umsetzung sind in Tabelle 3 dargestellt. Den Versuchsergebnissen ist zu entnehmen, daß bei einem hälftigen Isobutenumsatz bereits ca. 95% des anwesenden Butadien-(1,3) hydriert sind.

### Beispiel 9 (Vergleichsbeispiel)

Der Versuch wurde, wie in Beispiel 7 beschrieben, durchgeführt, nur mit dem Unterschied, daß ohne Methanol gearbeitet wurde. Aus Tabelle 3 sind alle Versuchsbedingungen und Stoffzusammensetzungen ersichtlich.

Tabelle 3

Oligomerisierung von Raffinat I in Gegenwart von Wasserstoff an metalldotierten Kationenaustauschern

Kontaktmenge: 120 ml ≙ 55,8 g
Versuchsbedingungen: p = 8 bar; T = 50—60°C; WHSV = 5,6; H$_2$ = ca. 10 l · h$^{-1}$

| Zusammensetzung | Einsatz | Beispiel 8 SPC 118 H$^+$/Pd | 9 (Vergleich) SPC 118 H$^+$ |
|---|---|---|---|
| i-Butan (Gew.-%) | 3,56 | 3,5 | 3,8 |
| n-Butan (Gew.-%) | 10,25 | 15,0 | 11,4 |
| i-Buten (Gew.-%) | 48,30 | 21,7 | 0,1 |
| n-Buten-(1) (Gew.-%) | 27,40 | 6,0 | 12,2 |
| tr.-, cis-Buten-(2) (Gew.-%) | 16,00 | 30,3 | 30,0 |
| Butadien-(1,3) (Gew.-%) | 0,40 | 0,02 | 0,14 |
| C$_8$-Kohlenwasserstoffe (Gew.-%) | 0,1 | 20,2 | 27,1 |
| C$_{12}$-Kohlenwasserstoffe (Gew.-%) | 0,1 | 2,5 | 12,2 |
| C$_{16}$-Kohlenwasserstoffe (Gew.-%) | 0,1 | 0,7 | 1,7 |
| Umsatz: i-Buten (%) | — | 48,7 | 99,8 |
| Umsatz: Butadien-(1,3) (%) | — | 95,0 | 65,0 |

Vergleichsbeispiel 10

Die Versuche wurden, wie in Beispiel 7 beschrieben, durchgeführt, jedoch wurde als Kohlenwasserstoffgemisch ein i-Buten-armes Raffinat II eingesetzt. Außerdem wurde dem Reaktionsgemisch kein Methanol zugeführt.
Versuchsbedingungen und Analysenergebnisse s. Tabelle 4.

Beispiel 11

Die Versuche wurden, wie in Beispiel 6 beschrieben, durchgeführt, jedoch wurde als Kohlenwasserstoffgemisch ein i-Buten-armes Raffinat II eingesetzt. Außerdem wurde dem Reaktionsgemisch kein Methanol zugeführt.
Versuchsbedingungen und Analysenergebnisse s. Tabelle 4.

Tabelle 4

Oligomerisierung von Raffinat II in Gegenwart von Wasserstoff an metalldotierten Kationenaustauschern

| | | |
|---|---|---|
| Kontaktmenge: | 120 ml $\approx$ 55,8 g | |
| Versuchsbedingungen: | p = 15 bar; T = 50°C; WHSV = 3; $H_2$ = 0,2 l · h⁻¹ | |

| Zusammensetzung | Einsatz | Beispiel 10 (Vergleich) SPC 118 H⁺ | 11 SPC 118 H⁺/Pd |
|---|---|---|---|
| Butane (Gew.-%) | 24,5 | 24,9 | 25,2 |
| i-Buten (Gew.-%) | 3,1 | 0,1 | < 0,1 |
| n-Buten-1 (Gew.-%) | 12,6 | 10,8 | 9,9 |
| Buten-(2) (Gew.-%) | 59,7 | 59,7 | 59,7 |
| Butadien-(1,3) (ppm) | 400 | 400 | 50 |
| Acetylene (ppm) | 10 | 10 | <10 |
| $C_8$-Kohlenwasserstoffe und höhere (Gew.-%) | 0,02 | 4,5 | 5,1 |
| Umsatz: i-Buten (%) | — | 96,8 | >99 |
| Umsatz: Butadien-(1,3) (%) | — | 0 | 87 |

## Patentansprüche

1. Verfahren zur katalytischen Umsetzung von i-Alkenen und zur weitgehenden Entfernung von Acetylen-Verbindungen und gegebenenfalls von Diolefinen durch katalytische Hydrierung in Kohlenwasserstoffströmen, die i-Alkene, Acetylen-Verbindungen und gegebenenfalls Diolefine enthalten, dadurch gekennzeichnet, daß man die katalytische Umsetzung und die katalytische Hydrierung gleichzeitig an einem makroporösen oder gelförmigen Kationenaustauscher in der H⁺-Form, der 0,001 bis 10 g eines oder mehrerer Metalle der VI. und/oder VII. und/oder der VIII. Nebengruppe des Periodensystems der Elemente in elementarer Form pro Liter trockenen Kationenaustauschers enthält und einen Vernetzungsgrad von 2 bis 65% sowie eine spezifische Oberfläche von 5 bis 750 m²/g trockenes Austauscherharz aufweist, in flüssiger Phase bei 20 bis 140°C durchführt.

2. Verfahren nach Anspruch 1, dadurch gekennzeichnet, daß der Kationenaustauscher als Metall Palladium, Platin, Rhenium, Molybdän oder Nickel jeweils für sich allein enthält.

3. Verfahren nach Anspruch 1 und 2, dadurch gekennzeichnet, daß man die Umsetzung bei einer Belastung des Kationenaustauschers von 0,1 bis 100 kg Kohlenwasserstoffstrom pro kg Kationenaustauscher pro Stunde durchführt.

4. Verfahren nach Anspruch 1 bis 3, dadurch gekennzeichnet, daß Wasserstoff in einer Menge von 0,001 bis 1 Mol pro Mol des Kohlenwasserstoffstroms eingesetzt wird.

5. Verfahren nach Anspruch 1 bis 4, dadurch gekennzeichnet, daß der Reaktionsdruck so eingestellt wird, daß bei der gewählten Arbeitstemperatur mindestens ein Teil der Einsatz- und Umsetzungsprodukte in flüssiger Phase vorliegt.

6. Verfahren nach Anspruch 1 bis 5, dadurch gekennzeichnet, daß man i-Alkene enthaltende Kohlenwasserstoffströme einsetzt, die beim thermischen oder katalytischen Cracken von Erdölfraktionen oder bei der Aufarbeitung bzw. Weiterverarbeitung solcher Crack-Kohlenwasserstoffströme anfallen.

7. Verfahren nach Anspruch 6, dadurch gekennzeichnet, daß die Kohlenwasserstoffströme neben i-Alkenen 0,001 bis 70 Vol-%, bezogen auf das Volumen des Gesamtstroms, an Butadien enthalten.

8. Verfahren nach Anspruch 1 bis 7, dadurch gekennzeichnet, daß als katalytische Umsetzung von i-Alkenen ihre Veretherung in Gegenwart von 0,1 bis 4 Mol Alkanol pro Mol i-Alkenen durchgeführt wird.

9. Verfahren nach Anspruch 8, dadurch gekennzeichnet, daß als Alkanol Methanol eingesetzt wird.

10. Verfahren nach Anspruch 1 bis 7, dadurch gekennzeichnet, daß als katalytische Umsetzung von i-Alkenen die Oligomerisierung von i-Buten durchgeführt wird.

**Claims**

1. Process for the catalytic reaction of i-alkenes and for the extensive removal of acetylene compounds and, if appropriate, diolefines by catalytic hydrogenation in hydrocarbon streams which contain i-alkenes, acetylene compounds und possibly diolefines, characterised in that the catalytic reaction and the catalytic hydrogenation are carried out simultaneously on a macroporous or gel-like cation exchanger in the $H^+$ form, which contains 0.001 to 10 g of one or more metals of the VIth and/or VIIth and/or VIIIth sub-group of the periodic system of the elements in an elementary form per litre of dry cation exchanger and which has a degree of crosslinking of 2 to 65% and a specific surface area of 5 to 750 $m^2$/g of dry exchanger resin, in the liquid phase at 20 to 140° C.

2. Process according to Claim 1, characterised in that the cation exchanger contains as the metal palladium, platinum, rhenium, molybdenum or nickel, each separately.

3. Process according to Claim 1 and 2, characterised in that the reaction is carried out with a loading of the cation exchanger of 0.1 to 100 kg of hydrocarbon stream per kg of cation exchanger per hour.

4. Process according to Claim 1 to 3, characterised in that hydrogen is employed in an amount of 0.001 to 1 mol per mol of the hydrocarbon stream.

5. Process according to Claim 1 to 4, characterised in that the reaction pressure is set up so that, at the selected working temperature, at least a part of the inlet and reaction products are present in the liquid phase.

6. Process according to Claim 1 to 5, characterised in that hydrocarbon streams, containing i-alkenes, are employed which result from thermal or catalytic cracking of petroleum oil fractions or by processing or further processing cracked hydrocarbon streams of these types.

7. Process according to Claim 6, characterised in that the hydrocarbon streams contain, apart from i-alkenes, 0.001 to 70% by volume, relative to the total volume of the stream, of butadiene.

8. Process according to Claim 1 to 7, characterised in that the catalytic reaction of i-alkenes which is carried out is the etherification thereof in the presence of 0.1 to 4 mols of alkanol per mol of i-alkenes.

9. Process according to Claim 8, characterised in that methanol is used as the alkanol.

10. Process according to Claim 1 to 7, characterised in that the catalytic reaction of i-alkenes which is carried out is the oligomerisation of i-butene.

**Revendications**

1. Procédé de transformation catalytique d'isoalcènes et d'élimination poussée de composés acétyléniques et, le cas échéant, de dioléfines par hydrogénation catalytique dans des courants d'hydrocarbures qui contiennent des iso-alcènes, des composés acétyléniques et, le cas échéant, des dioléfines, caractérisé en ce qu'on conduit la réaction catalytique et l'hydrogénation catalytique simultanément en phase liquide à 20—140° C sur un échangeur cationique macroporeux ou gélifié sous la forme $H^+$, qui contient 0,001 à 10 g d'un ou plusieurs métaux des sousgroupes VI et/ou VII et/ou du sous-groupe VIII du Système Périodique des Eléments sous la forme élémentaire, par litre d'échangeur cationique sec et présente un degré de réticulation de 2 à 65% ainsi qu'une surface spécifique de 5 à 750 $m^2$/g de résine échangeuse sèche.

2. Procédé suivant la revendication 1, caractérisé en ce que l'échangeur cationique contient comme métal du palladium, du platine, du rhénium, du molybdène ou du nickel, individuellement dans chaque cas.

3. Procédé suivant les revendications 1 et 2, caractérisé en ce qu'on conduit la transformation avec une charge de l'échangeur cationique de 0,1 à 100 kg de courant d'hydrocarbures par kg d'échangeur cationique par heure.

4. Procédé suivant les revendications 1 à 3, caractérisé en ce que de l'hydrogène est utilisé en une quantité de 0,001 à 1 mole par mole de courant d'hydrocarbures.

5. Procédé suivant les revendications 1 à 4, caractérisé en ce qu'on règle la pression de réaction de manière qu'au moins une partie des produits chargés et des produits de transformation se présente sous la forme liquide à la température de travail choisie.

6. Procédé suivant les revendications 1 à 5, caractérisé en ce qu'on utilise des courants d'hydrocarbures contenant des iso-alcènes qui sont obtenus lors du craquage thermique ou catalytique de fractions de pétrole ou lors du traitement ou de la transformation ultérieure de ces courants d'hydrocarbures de craquage.

7. Procédé suivant la revendication 6, caractérisé en ce que les courants d'hydrocarbures contiennent 0,001 à 70% en volume, par rapport au volume du courant total, de butadiène à côte des iso-alcènes.

8. Procédé suivant les revendications 1 à 7, caractérisé en ce qu'on effectue comme transformation catalytique d'iso-alcènes leur éthérification en présence de 0,1 à 4 moles d'alcanol par mole d'iso-alcènes.

9. Procédé suivant la revendication 8, caractérisé en ce qu'on utilise comme alcanol le méthanol.

10. Procédé suivant les revendications 1 à 7, caractérisé en ce qu'on conduit comme transformation catalytique d'iso-alcènes l'oligomérisation de l'isobutène.